Europäisches Patentamt

European Patent Office

Office européen des brevets

(11) Veröffentlichungsnummer: **0 111 723**
**B1**

(12) **EUROPÄISCHE PATENTSCHRIFT**

(45) Veröffentlichungstag der Patentschrift:
27.08.86

(21) Anmeldenummer: 83111142.2

(22) Anmeldetag: 08.11.83

(51) Int. Cl.⁴: **A 61 M 5/14**

(54) Zuspritzvorrichtung für ein Infusions- oder Transfusionssystem.

(30) Priorität: **21.12.82 DE 8235862 U**

(43) Veröffentlichungstag der Anmeldung:
**27.06.84 Patentblatt 84/26**

(45) Bekanntmachung des Hinweises auf die Patenterteilung:
**27.08.86 Patentblatt 86/35**

(84) Benannte Vertragsstaaten:
**AT BE CH DE FR GB IT LI LU NL SE**

(56) Entgegenhaltungen:
**DE - A - 1 949 038
DE - A - 3 020 449
DE - C - 2 401 782
DE - C - 2 458 405
GB - A - 2 006 035
US - A - 4 219 912
US - A - 4 261 474**

(73) Patentinhaber: **INTERMEDICAT GMBH,
Gerliswilstrasse 74, CH-6020 Emmenbrücke (CH)**

(72) Erfinder: **Herlitze, Gerhard, Binsdorfer Strasse 4,
D-3507 Baunatal 7 (DE)**

(74) Vertreter: **von Kreisler, Alek, Dipl.-Chem. et al,
Deichmannhaus am Hauptbahnhof, D-5000 Köln 1 (DE)**

## Beschreibung

Die Erfindung betrifft eine Zuspritzvorrichtung für ein Infusions- oder Transfusionssystem, mit einem Gehäuse, welches einen einseitig durch eine durchstechbare Membran abgeschlossenen Kanal aufweist, in dem mit Abstand hinter der Membran ein Partikelfilter aus einem Sinterkörper angeordnet ist.

Es ist bekannt, in die Schlauchleitung, die von einer Tropfkammer zu einem Patienten führt, ein flexibles Latex-Schlauchstück einzusetzen, um der Infusions- oder Transfusionslösung flüssige Medikamente o. dgl. zuspritzen zu können. Hierzu wird das Latex-Schlauchstück mit der Kanüle einer Spritze durchstochen und aus der Spritze wird das Injektat in die zum Patienten führende Leitung injiziert. Anschliessend kann die Spritzenkanüle wieder aus dem Latex-Schlauchstück herausgezogen werden, das infolge seiner Elastizität die Perforationsstelle selbsttätig wieder schliesst.

Bekannt sind ferner Zuspritzgehäuse, die nach Art von Verschlussstopfen ausgebildet sind und zum Verschliessen der Öffnung eines Anschlussstückes des Infusions- oder Transfusionssystems benutzt werden. Diese Verschlussstopfen haben einen durchgehenden Kanal, der am äusseren Ende mit einer durchstechbaren Membran abdichtend verschlossen ist. Zum Zuspritzen eines Medikamentes o. dgl. wird eine Spritzenkanüle durch die Membrane hindurchgestochen. Beim Ausdrücken der Spritze gelangt das Medikament in den Kanal des Gehäuses und anschliessend in die zum Patienten führende Leitung.

Es hat sich herausgestellt, dass das Injektat in vielen Fällen Partikel enthält, die aus der Spritze selbst oder aus der Glasampulle stammen, aus der die Spritze aufgezogen worden ist. Ausserdem entsteht beim Durchstechen der Spritzenkanüle durch die Membran der Zuspritzkanüle häufig ein Abrieb an der Membran, wodurch Partikel in den zum Patienten führenden Kanal gelangen. Auch Ausstanzungen, die von der Metallkanüle an der Membran vorgenommen werden, gelangen in das patientenseitige Flüssigkeitssystem. Solche Partikel bilden eine ernsthafte Gefährdung für den Patienten, da sie zu Verstopfungen im Blutsystem führen können. Es ist bekannt, in Flüssigkeitssystemen, die an einen Patienten angeschlossen sind, mikrofeine Filter zu verwenden, die den Durchgang der Flüssigkeit zulassen, Partikel aber herausfiltern. Derartige Feinfilter bestehen in der Regel aus einem oder mehreren engmaschigen Fasergeweben. Die mechanische Festlegung derartiger Filter ist jedoch sehr schwierig. Die Filter haben darüber hinaus eine relativ kleine wirksame Filterfläche, so dass ihre Wirksamkeit und Durchlässigkeit nach dem Auffangen von Partikeln stark nachlässt.

Eine aus DE-A-2458405 bekannte Zuspritzvorrichtung der eingangs genannten Art weist ein Gehäuse auf, das einen zylindrischen Filterträger mit darauf befestigtem zylindrischem Filterelement enthält. An einem Ende des Gehäuses sind zwei seitliche Zuspritzeinlässe vorgesehen, von denen jeder mit einer durchstechbaren Membran verschlossen ist. Die Membranen sind seitlich angeordnet, damit die eingeführte Injektionsnadel das unbenetzbare Filterelement nicht erreichen und beschädigen kann. Die bekannte Zuspritzvorrichtung besteht aus zahlreichen Teilen, ist schwer zu montieren und benötigt viel Platz.

Ein aus DE-A-2401782 bekannter Filter, der nicht in eine Zuspritzvorrichtung integriert ist, enthält einen Sinterkörper. Die Befestigung des Sinterkörpers im Gehäuse erfolgt mit Presssitz an einem ringförmigen Wulst. Durch induktive Beheizung wird der gesamte Sinterkörper erwärmt, wodurch der Wulst des Gehäuses zum Schmelzen gebracht wird und das Gehäusematerial in die Randzonen des Filterkörpers eindringt und dort die Poren füllt.

Bei einem weiteren bekannten Filter nach DE-A-3020449 ist ein Sinterkörper ebenfalls mit Presssitz in einem rohrförmigen Gehäuse festgehalten. Eine Ringschulter des Gehäuses sorgt für die Positionierung des Filterkörpers in Richtung zum Patienten, während in Gegenrichtung keine definierte Festlegung erfolgt. In dem beschriebenen Infusionssystem ist im Zuge der zum Patienten führenden Schlauchleitung vor dem Filter eine separate Zuspritzvorrichtung vorhanden.

Der Erfindung liegt die Aufgabe zugrunde, eine Zuspritzvorrichtung zu schaffen, die einfach im Aufbau ist und eine einfache und sichere Befestigung des Filters ermöglicht.

Die Lösung dieser Aufgabe besteht erfindungsgemäss darin, dass die Membran an der Stirnwand des Gehäuses befestigt ist, dass der zwischen der Membran und dem Sinterkörper liegende Abschnitt des Kanals eine grössere Weite hat als der hinter dem Sinterkörper liegende Abschnitt und dass der Sinterkörper in einen passenden ringförmigen Stufenabschnitt des Gehäuses eingesetzt und an seinem der Membran zugewandten Ende durch einen umgebogenen Rand am Ende des Stufenabschnitts umgriffen ist.

Der Sinterkörper bildet einen Tiefenfilter mit einem Filtervolumen, in welchem Partikel zurückgehalten werden. Ein derartiger Tiefenfilter hat eine sehr grosse Filterfläche bzw. ein grosses Filtervolumen, so dass die Gefahr von Verstopfungen im Vergleich zu Flächenfiltern gering ist. Da der Sinterkörper insgesamt starr und fest ist, kann er von der Injektionskanüle nicht versehentlich durchstochen werden. Die Spitze der Injektionskanüle bleibt also in jedem Fall in dem Raum zwischen der Membran und dem Sinterkörper. Aus diesem Raum heraus können Partikel nicht in die patientenseitigen Flüssigkeitsleitungen eindringen.

Ein weiterer Vorteil der erfindungsgemässen Zuspritzvorrichtung besteht darin, dass der Filterkörper auf einfache Weise in dem Gehäuse der Zuspritzvorrichtung festgelegt wird. Der Sinterkörper fügt sich formschlüssig in den Stufenabschnitt des Gehäuses ein, und er wird durch den umgebogenen Rand sicher festgehalten. Zusätzliche Abdichtungen oder separate Befestigungsmittel sind nicht erforderlich. Die gesamte Zuspritzvorrichtung kann somit aus lediglich drei Teilen gefertigt werden, nämlich dem aus Kunststoff bestehenden Gehäuse, der Membran und dem Sinterkörper.

Der Sinterkörper besteht vorzugsweise aus einem Polymerwerkstoff, also aus Kunststoff. Er kann aber auch aus einem Sintermetall bestehen.

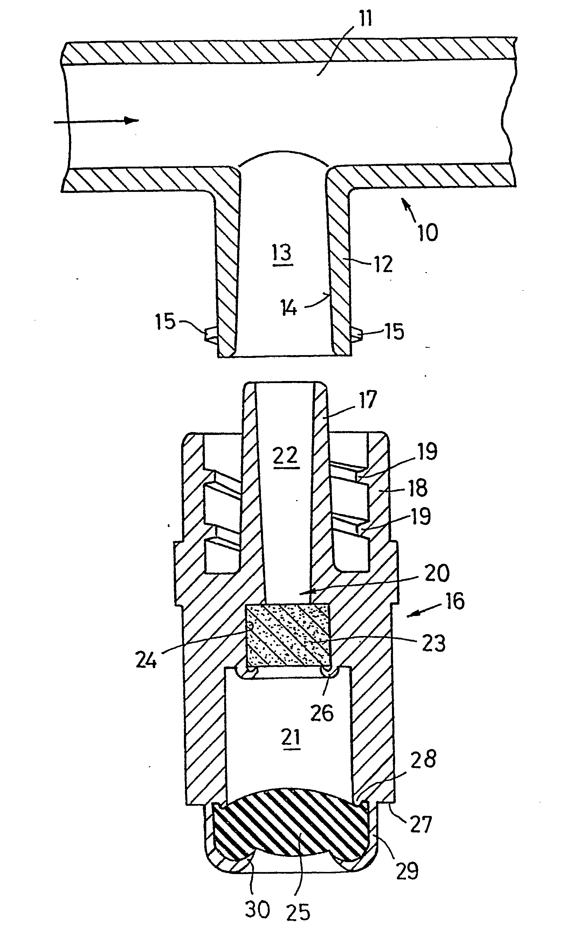

Im folgenden wird unter Bezug auf die einzige Figur der Zeichnung ein Ausführungsbeispiel der Erfindung näher erläutert.

Die Zeichnung zeigt einen Längsschnitt durch die Zuspritzvorrichtung. Ein Verbindungsstück 10, das einen zum Patienten führenden Überleitungskanal 11 aufweist und aus starrem Kunststoff besteht, ist in die von einer Tropfkammer zum Patienten führende Schlauchleitung eingesetzt. Das Verbindungsstück 10 weist einen Anschlussstutzen 12 auf, welcher einen von dem Überleitungskanal 11 abgehenden Zweigkanal 13 mit sich nach aussen konisch erweiternder Innenwand 14 enthält. Der Abzweigstutzen 12 ist als Luer-Innenkonus ausgebildet und weist an seiner Aussenseite abstehende Verriegelungselemente 15 auf.

Die Zuspritzvorrichtung 16, die mit dem Anschlussstutzen 12 abdichtend verbunden wird, ist mit einem Aussenkonus 17 versehen, der passend und abdichtend in den Innenkonus des Anschlussstutzens 12 einsetzbar ist. Der Aussenkonus 17 ist mit radialem Abstand von einer Muffe 18 umgeben, welche an ihrer Innenseite Gewindeteile 19 aufweist, die mit den Verriegelungselementen 15 des Anschlussstutzens 12 zusammenwirken. Der Kanal 20, der sich in Längsrichtung durch das Gehäuse 16 erstreckt, besteht aus einem ersten Abschnitt 21 und einem sich durch den Anschlusskonus 17 erstreckenden zweiten Abschnitt 22. Die beiden Abschnitte 21 und 22 sind durch den porösen Sinterkörper 23 voneinander getrennt. Der Sinterkörper 23 sitzt passend in einem Stufenabschnitt des Gehäuses 16. Der Durchmesser des Stufenabschnitts 24 ist grösser als der Durchmesser des Abschnitts 22 und kleiner als der Durchmesser des Abschnitts 21. Zur Festlegung des Sinterkörpers 23 ist der der durchstechbaren Membran 25 zugewandte Rand 26 des Stufenabschnitts 24 umgebogen, so dass er den Rand des Sinterkörpers 23 unter Bildung eines axial vorstehenden Ringwulstes umschliesst. Hierdurch wird der Sinterkörper 23 in dem Stufenabschnitt 24 festgehalten. Das Gehäuse 16 ist einstückig aus einem thermoplastischen Kunststoff gefertigt und der Rand 26 ist unter Warmverformung umgebogen.

Der Abschnitt 21, der an seinem einen Ende durch den Sinterkörper 23 begrenzt wird, wird an seinem äusseren Ende durch die aus Latex bestehende durchstechbare Membran 25 abgeschlossen. Die Membran 25 ist an dem Ende des Gehäuses 16 befestigt. An der Innenkante der ringförmigen Stirnwand 27 des Gehäuses 16 befindet sich ein axial vorstehender Ringwulst 28, gegen den die Membran 15 gesetzt ist. Der Ringwulst 28 ist mit radialem Abstand von einem einstückig mit dem Gehäuse 16 verbundenen Ring 29 umgeben, der die Membran 25 radial umschliesst und dessen Rand 30 halbkreisförmig nach innen umgebogen ist, so dass seine äussere Kante in die Aussenseite der Membran 25 eindrückt. Auf diese Weise wird die Membran 25 in der dargestellten Weise verformt und der von dem Rand 30 umschlossene Bereich der Membran 25 kann mit einer (nicht dargestellten) Injektionskanüle durchstochen werden. Wenn die Spitze der Injektionskanüle in den Abschnitt 21 des Kanals 20 eingedrungen ist, wird das Injektat in den Abschnitt 21 injiziert. Von dort gelangt es durch den Sinterkörper 23 hindurch in den Abschnitt 22 und von diesem in den Überleitungskanal 11.

Für verschiedene Anwendungen ist es wichtig, sicherzustellen, dass das Gehäuse 16 nicht unbeabsichtigt von dem Verbindungsstück 10 gelöst werden kann. Um dies zu erreichen, kann das Gehäuse 16 an dem Anschlussstutzen 12 festgeklebt werden, so dass es mit dem Verbindungsstück 10 eine nicht lösbare Baueinheit bildet, wobei der Kanal 20 stets mit dem Zweigkanal 13 bzw. dem Überleitungskanal 11 in Verbindung steht. Alternativ kann das Gehäuse 16 mit dem Verbindungsstück 10 einstückig gefertigt werden, so dass der Filterkörper 23 und die Membran 25 fest und dauerhaft mit dem Verbindungsstück verbunden sind. Auf diese Weise wird mit Sicherheit verhindert, dass Luft in den Überleitungskanal 11 eindringen kann.

## Patentansprüche

1. Zuspritzvorrichtung für ein Infusions- oder Transfusionssystem, mit einem Gehäuse, welches einen einseitig durch eine durchstechbare Membran (25) abgeschlossenen Kanal (20) aufweist, in dem mit Abstand hinter der Membran (25) ein Partikelfilter aus einem Sinterkörper (23) angeordnet ist, dadurch gekennzeichnet, dass die Membran (25) an der Stirnwand (27) des Gehäuses befestigt ist, dass der zwischen der Membran (25) und dem Sinterkörper (23) liegende Abschnitt (21) des Kanals (20) eine grössere Weite hat als der hinter dem Sinterkörper (23) liegende Abschnitt (22) und dass der Sinterkörper (23) in einen passenden ringförmigen Stufenabschnitt (24) des Gehäuses (16) eingesetzt und an seinem der Membran (25) zugewandten Ende durch einen umgebogenen Rand (26) am Ende des Stufenabschnitts (24) umgriffen ist.

2. Zuspritzvorrichtung nach Anspruch 1, dadurch gekennzeichnet, dass der Sinterkörper (23) aus einem Polymerwerkstoff besteht.

3. Zuspritzvorrichtung nach Anspruch 1, dadurch gekennzeichnet, dass der Sinterkörper (23) aus einem Sintermetall besteht.

## Claims

1. Injection site device for an infusion or transfusion system comprising a housing having a channel (20) closed at one end by a pierceable membrane (25) and including a particle filter of a sintered body arranged at a distance behind the membrane (25), characterized in that the mem-

**0111723**

brane (25) is fixed at the end wall (27) of the housing, that the section (21) of the channel (20) situated between the membrane (25) and the sintered body (23) is broader than the section (22) situated behind the sintered body (23) and that the sintered body (23) is mounted to fit into an annular stepped portion (24) of the housing (16) in being overengaged at the end of the stepped portion (24) by a folded border (26) at its end confronted with the membrane (25).

2. Injection site device as defined in claim 1, characterized in that the sintered body is made of a polymeric material.

3. Injection site device according to claim 1, characterized in that the sintered body (23) consists of a dry powered metal.

**Revendications**

1. Dispositif d'injection pour un système de perfusion ou de transfusion, comportant un boîtier, qui possède un canal (20) fermé d'un côté par une membrane (25) pouvant être perforée et dans lequel se trouve disposé, derrière la membrane (25), et à distance de cette dernière, un filtre de retenue des particules, constitué par un corps fritté (23), caractérisé en ce que la membrane (25) est fixée sur la paroi frontale (27) du boîtier, que la section (21) du canal (20), qui est située entre la membrane (25) et le corps fritté (23), possède une largeur supérieure à celle de la section (22) située en aval du corps fritté (23), et que le corps fritté (23) est inséré dans une section annulaire étagée appropriée (24) du boîtier (16) et est enserré, sur son extrémité tournée vers la membrane (25), par un bord recourbé (26) situé au niveau de l'extrémité de la section étagée (24).

2. Dispositif d'injection selon la revendication 1, caractérisé en ce que le corps fritté (23) est constitué par un matériau polymère.

3. Dispositif d'injection selon la revendication 1, caractérisé en ce que le corps fritté (23) est constitué par un métal fritté.